Europäisches Patentamt

European Patent Office

Office européen des brevets

(11) Veröffentlichungsnummer: **0 348 016**
**A2**

(12) **EUROPÄISCHE PATENTANMELDUNG**

(21) Anmeldenummer: 89201966.2

(22) Anmeldetag: 30.03.88

(51) Int. Cl.⁴: **A61B 17/22**

(30) Priorität: 04.04.87 DE 3711404

(43) Veröffentlichungstag der Anmeldung:
**27.12.89 Patentblatt 89/52**

(60) Veröffentlichungsnummer der früheren
Anmeldung nach Art. 76 EPÜ: **0 286 170**

(84) Benannte Vertragsstaaten:
**DE FR GB**

(71) Anmelder: **Philips Patentverwaltung GmbH**
**Wendenstrasse 35 Postfach 10 51 49**
**D-2000 Hamburg 1(DE)**
(84) **DE**

Anmelder: **N.V. Philips' Gloeilampenfabrieken**
**Groenewoudseweg 1**
**NL-5621 BA Eindhoven(NL)**
(84) **FR GB**

Anmelder: **DORNIER MEDIZINTECHNIK GMBH**
**Industriestrasse 15**
**D-8034 Germering(DE)**
(84) **DE FR GB**

(72) Erfinder: **Schwieker, Horst**
**Trenknerweg 21**
**D-2000 Hamburg 52(DE)**
Erfinder: **Artmeier, Theo**
**Kennedystrasse 10**
**D-8039 Puchheim(DE)**

(74) Vertreter: **Hartmann, Heinrich, Dipl.-Ing. et al**
**Philips Patentverwaltung GmbH**
**Wendenstrasse 35 Postfach 10 51 49**
**D-2000 Hamburg 1(DE)**

(54) **Lithotripsie-Arbeitsplatz.**

(57) Die Erfindung betrifft einen Lithotripsie-Arbeitsplatz mit einer Röntgeneinrichtung zur Ortung Konkrementen und einem Stoßwellenerzeuger zum Zertrümmern der Konkremente. Der Stoßwellenerzeuger kann um eine zur Tischlängsrichtung parallele Achse aus dem Strahlengang der Röntgeneinrichtung geschwenkt werden.

Fig.1

EP 0 348 016 A2

## Lithotripsie-Arbeitsplatz

Die Erfindung betrifft einen Lithotripsie-Arbeitsplatz nach dem Oberbegriff des Anspruchs 1. Aus der EP-A 205 878 ist bereits ein Lithotripsie-Arbeitsplatz bekannt mit einer einen Röntgenstrahler und einen darauf zentrierten Bildwandler umfassenden Röntgeneinrichtung, die an einem C-förmigen Träger befestigt ist, welcher die Patientenliegen seitlich umgreift und um zwei horizontale Achsen schwenkbar ist, von denen eine parallel zur Tischlängsrichtung und die andere senkrecht dazu verläuft. Bei dem bekannten Gerät ist der Stoßwellenerzeuger ebenfalls an einem C-förmigen Träger befestigt, welcher seinerseits mit einem auf dem Boden fahrbaren Wagen verbunden ist.

Bei einer Röntgenuntersuchung - beispielsweise zur Ortung eines Nierensteins - muß der Wagen aus dem Strahlengang gefahren werden, um zu verhindern, daß der daran befestigte C-förmige Träger für den Stoßwellenerzeuger den Strahlengang unterbricht. Wenn dann der Nierenstein geortet worden ist, muß der auf dem Boden verfahrbare Wagen wieder in seine Therapiestellung zurückgebracht werden. Dabei müssen Vorkehrungen getroffen werden, um sicherzustellen, daß der Fokuspunkt F des Stoßwellenerzeugers auf den zuvor georteten Nierenstein ausgerichtet wird.

Aufgabe der vorliegenden Erfindung ist es, einen Lithotripsie-Arbeitsplatz der eingangs genannten Art so auszugestalten, daß mit einfachen Mitteln der Stoßwellenerzeuger nach seiner Entfernung aus dem Strahlengang wieder in den Strahlengang zurückbewegt werden kann derart, daß der Fokus des Stoßwellenerzeugers ohne weiteres auf den Schnittpunkt des Zentrahlstrahls mit der ersten Achse ausgerichtet werden kann, so daß die Energie des Stoßwellenerzeugers auf ein dort (nach vorheriger Ortung des Konkrementes und Positionierung des Patienten) befindliches Konkrement konzentriert werden kann. Diese Aufgabe wird durch die im Kennzeichen des Anspruchs 1 angegebenen Maßnahmen gelöst.

Bei der Erfindung wird also der Stoßwellenerzeuger dadurch aus dem Strahlengang (und wieder zurück) bewegt, daß der Lagerhalter, der den Schwenkarm mit dem Stoßwellenerzeuger trägt, um die dritte, zur Tischlängsrichtung parallele Achse geschwenkt wird. Es ist auf diese Weise sehr einfach und genau möglich, den Stoßwellenerzeuger durch Schwenken des Lagerhalters um die dritte Achse wieder in die Position bringen, in der der Fokus mit dem Schnittpunkt des Zentralstrahls mit der ersten Achse zusammenfällt. Auch wenn dann der Schwenkarm um die zweite Achse nach links oder rechts geschwenkt wird, bleibt diese Ausrichtung erhalten.

Die Erfindung wird nachstehend anhand der Zeichnung näher erläutert. Es zeigen:

Fig. 1 eine Seitenansicht eines Arbeitsplatzes nach der Erfindung,

Fig. 2 eine Frontansicht eines solchen Arbeitsplatzes bei geänderter Stellung des Stoßwellenerzeugers und

Fig. 3 einen Ausschnitt aus dieser Ansicht mit verschiedenen Stellungen des Stoßwellenerzeugers.

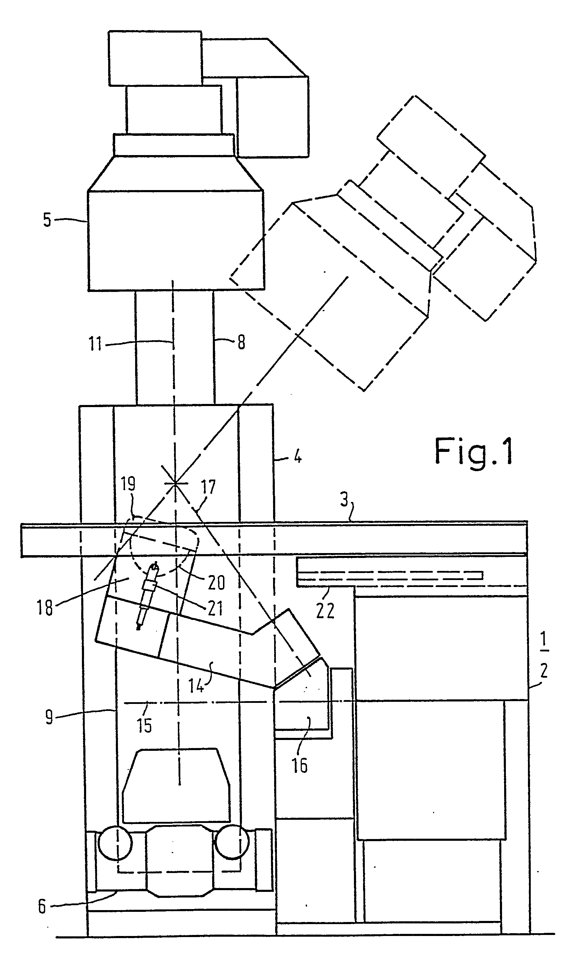

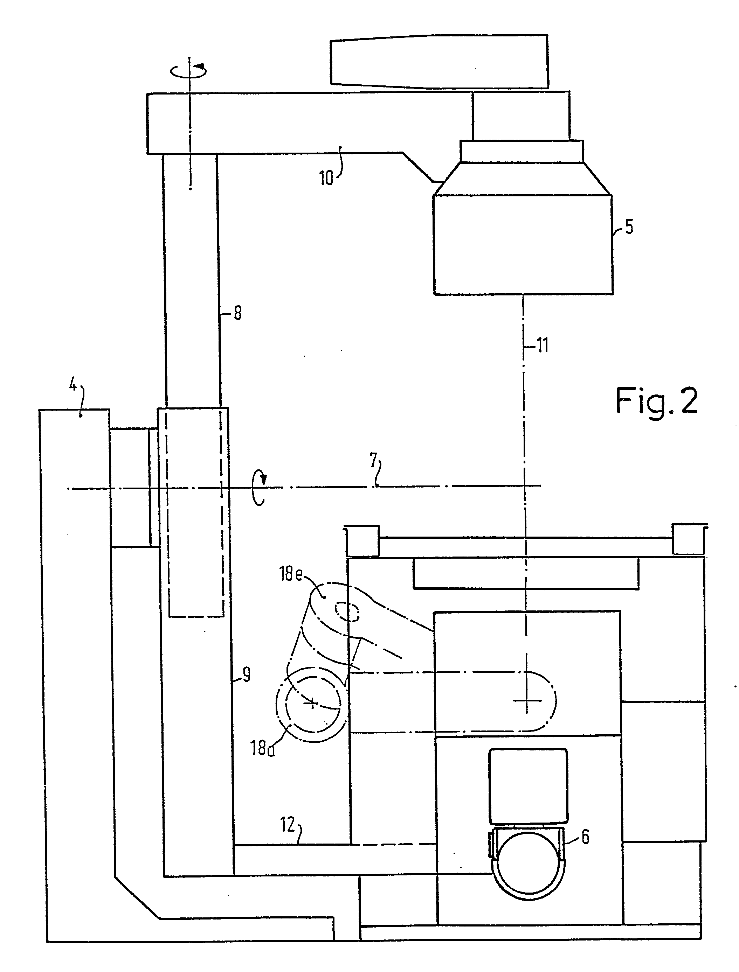

In den Fig. 1 und 2 ist mit 1 ein Patientenlagerungstisch mit einem Sockel 2 und einer Tischplatte 3 bezeichnet, die in ihrer längsrichtung (in Fig. 1 horizontal), in Querrichtung (senkrecht zur Zeichenebene der Fig. 1) und in der Höhe verstellbar ist. An einem Stativ 4 ist eine Röntgeneinrichtung um eine horizontale, zur Tischlängsrichtung senkrechte Achse 7 schwenkbar angeordnet. Die Röntgeneinrichtung umfaßt einen Röntgenstrahler 6, der an einem Träger 12 am Ende eines um die Achse 7 schwenkbaren Hohlarmes 9 befestigt ist, und einen Röntgenbildverstärker 5. Der Röntgenbildverstärker ist an einem Träger 10 befestigt, der seinerseits am Ende eines Armes 8 um dessen Längsachse schwenkbar befestigt ist. Der Arm 8 kann innerhalb des Hohlarms 9 verschoben werden, so daß der Abstand zwischen dem Röntgenbildverstärker 5 und dem Röntgenstrahler 6 geändert werden kann.

Unter der Tischplatte befindet sich ein um eine horizontale, zur Tischlängsrichtung parallel verlaufende Achse 15 schwenkbarer Lagerhalter 16, in dem ein Schwenkarm 14 um eine Achse 17 schwenkbar gelagert ist, die mit der Achse 15 einen Winkel von ca. 55° einschließt. Am freien Ende des Schwenkarms 14 ist ein Stoßwellengenerator 18 befestigt. Der Stoßwellengenerator enthält einen mit Wasser gefüllten, durch eine Membran 19 abgeschlossenen Hohlraum in Form eines Rotationsellipsoids 20, in dessen einem Brennpunkt sich eine Funkenstrecke 21 zur Erzeugung einer Stoßwelle befindet. Der andere Brennpunkt des Rotationsellipsoids - im folgenden Fokuspunkt genannt - liegt auf der Schwenkachse 17. In ihm konzentriert sich in bekannter Weise die durch die Stoßwelle erzeugte Energie. Die Energie zur Steinzertrümmerung kann auch auf andere Weise erzeugt werden, beispielsweise dadurch, daß auf einem geeignet geformten Rotationskörper Ultraschallwandler angeordnet sind, deren Energie in einem Punkt fokussiert wird.

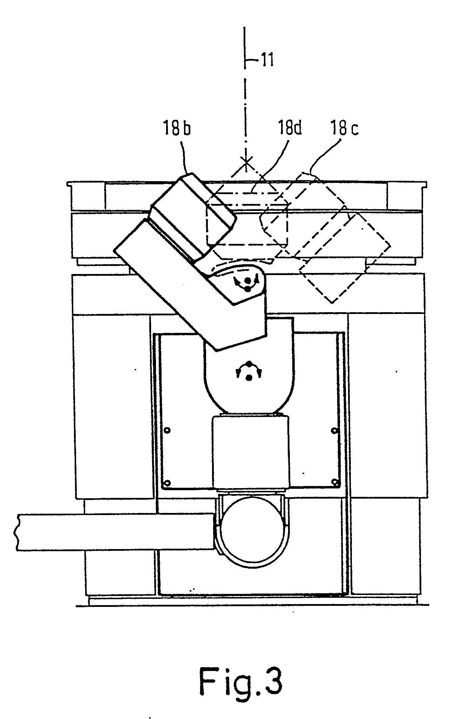

Wenn der Lagerhalter sich in der Position (vgl. Fig. 1 und Fig. 3) befindet, in der die Schwenkachse 17 mit der Schwenkachse 15 eine vertikale, zur Tischlängsrichtung parallele Ebene definiert, fällt der Fokuspunkt mit dem Schnittpunkt des Zentralstrahls und der horizontalen Schwenkachse 7 zusammen. Wenn der Patient zuvor so positioniert ist, daß der zu zertrümmernde Stein sich in diesem Schnittpunkt befindet, kann in dieser Stellung des Lagerhalters der Schwenkarm 14so um die Achse 17 geschwenkt werden, bis eine im Hinblick auf die Anatomie des Patienten optimale Position erreicht ist, ohne daß dadurch die Fokussierung beeinträchtigt wird. Der beschriebene Arbeitsplatz ist nicht nur in Verbindung mit der Lithotripsie verwendbar, sondern auch für urologische oder andere Röntgenuntersuchungen - wie im folgenden erläutert wird.

Zur Anfertigung von Untertischaufnahmen wird der Stoßwellenerzeuger 18 aus dem Strahlengang geschwenkt. Dies kann beispielsweise dadurch erfolgen, daß der Lagerhalter 16 um die Achse 15 so geschwenkt wird, daß die Achsen 15 und 17 eine horizontale Ebene definieren und daß der Schwenkarm um die Achse 17 um 180° geschwenkt wird. Er nimmt dann die in Fig. 2 gestrichelt angedeutete und mit 18a bezeichnete Parkstellung ein.

Es ist aber auch möglich, Röntgenuntersuchungen mit umgekehrtem Strahlengang durchzuführen. Zu diesem Zweck wird zunächst der Arm 8 ganz in den Hohlarm 9 hineingefahren, der Träger 10 um den Arm 8 geschwenkt und dann die Röntgeneinrichtung um 180° um die Achse 7 geschwenkt, wobei sich der Röntgenstrahler um das Fußende herum nach oben und der Bildverstärker in dem Raum zwischen dem Tisch 1 und dem Stativ 4 nach untenbewegt. Dort wird der Träger 10 wieder zurückgeschwenkt, so daß der Röntgenstrahler 6 und der Bildverstärker 5 wieder aufeinander zentriert sind.

In dieser Stellung der Röntgenröhre können auch Buckyaufnahmen mit Hilfe einer unter der Tischplatte 3 angeordneten, in Tischlängsrichtung verfahrbaren Kassettenlade 22 angefertigt werden.

Zur Ortung von Konkrementen im menschlichen Körper, vorzugsweise von Nierensteinen, wird der Patient zunächst in der in Fig. 1 mit ausgezogenen Linien dargestellten Stellung der Röntgeneinrichtung durchstrahlt und nach einer Schwenkung der Röntgeneinrichtung um die Achse 7 (die in Fig. 1 für den Bildwandler 5 gestrichelt angedeutet ist) erneut aus der so veränderten Perspektive. Dies macht es möglich, die räumliche Lage des zu zertrümmernden Steins zu bestimmen und die Tischplatte so zu verschieben, daß sich der Stein im Schnittpunkt der horizontalen Achse 7 mit dem Zentralstrahl 11 befindet.

Danach wird der Lagerhalter 16 so geschwenkt, daß die Schwenkachsen 15 und 17 wieder eine vertikale Ebene definieren. In dieser Stellung des Lagerhalters 16 ist der Stoßwellenerzeuger 18 unabhängig von der Stellung des Schwenkarms 14 stets auf den Schnittpunkt der Schwenkachse 7 mit dem Zentralstrahl 11 fokussiert, und er bleibt es auch, wenn der Schwenkarm in die für die Steinzertrümmerung optimale Position geschwenkt wird.

Bei der Zertrümmerung eines Steins in der rechten Niere wird der Stoßwellenerzeuger - wie in Fig. 3 mit ausgezogenen Linien angedeutet und mit 18b bezeichnet - nach links geschwenkt, bis die Wirkungslinie des Stoßwellenerzeugers zusammen mit der horizontalen Schwenkachse zumindest annähernd eine vertikale, zur Tischlängsrichtung senkrechte Ebene definieren. Die Wirkungsrichtung verläuft dann etwa unter einem Winkel von 43° in bezug auf die Horizontale. Bei der Zertrümmerung eines Steins in der linken Niere wird der Stoßwellenerzeuger analog nach rechts geschwenkt (gestrichelte Position 18c) und bei einer Untersuchung eines Gallensteins in eine mittlere Position (18d bzw. Fig. 1).

In all diesen Fällen muß die Membran 19 des Stoßwellenerzeugers 18 unmittelbaren Kontakt mit dem Körper des Patienten haben; dementsprechend muß eine nicht näher dargestellte Öffnung in der Tischplatte 3 vorhanden sein.

Bei einem Stoßwellenerzeuger, bei dem die Stoßwelle mittels einer Funkenstrecke erzeugt wird, muß in regelmäßigen Abständen ein Elektrodenwechsel vorgenommen werden. Zu diesem Zweck wird aus der in Fig. 1 bzw. Fig. 3 (Position 18d) dargestellten Position der Schwenkarm 14 um 90° um die Achse 17 und der Lagerhalter 16 um 90° um die Achse 15 geschwenkt, so daß der Stoßwellenerzeuger die in Fig. 2 mit 18e bezeichnete Position einnimmt, bei der die Öffnung des Rotationselipsoids 20 im Stoßwellenerzeuger 18 nach unten gerichtet ist. In dieser Stellung kann die Funkenstrecken-Elektrode 21 entnommen werden, ohne daß die Wasserfüllung in dem durch den Rotationselipsoid 20 und die Membran 19 abgebildeten Raum verloren geht.

## Ansprüche

Lithotripsie-Arbeitsplatz mit einem Patientenlagerungstisch (1...3), einer einen Röntgenstrahler (6) und einen darauf zentrierten Bildwandler (5) umfassenden Röntgeneinrichtung zur Ortung von Konkrementen, die um eine erste, zur Tischlängsrichtung senkrechte, horizontale Achse (7) schwenkbar ist, die den Zentralstrahl in einem Schnittpunkt schneidet, und mit einem unterhalb der Tischplatte befindlichen Stoßwellenerzeuger (18), der an einem Schwenkarm (14) befestigt ist, der um eine zweite, durch den Fokuspunkt des Stoßwellenerzeugers (18) verlaufende Achse (17) schwenkbar an einem unterhalb des Patientenlagerungstisches (1..3) befindlichen Lagerhalter (16) angelenkt ist, der um eine dritte Achse (15) schwenkbar ist, wobei in der einen Schwenkstellung des Lagerhalters (16) die schräg verlaufende zweite Achse (17) mit der Tischlängsrichtung eine senkrechte Ebene definiert und der Fokuspunkt und der Schnittpunkt zusammenfallen, dadurch gekennzeichnet, daß die dritte Achse parallel zur Tischlängsrichtung verläuft.

Fig.1

Fig.2

Fig.3

3-Ⅲ-PHD 87-075A EP